# EUROPEAN PATENT APPLICATION

(11) **EP 3 735 961 A1**
(43) Date of publication of application: **11.11.2020**
(21) Application number: 18898824.0
(22) Date of filing: 21.12.2018
(51) Int. Cl.: A61K 8/90, A61K 8/25, A61K 8/29, A61K 8/58, A61K 8/02, A61Q 17/04

(54) **COSMETIC POWDER COMPOSITE AND COSMETIC COMPOSITION CONTAINING SAME**

(30) Priority: 04.01.2018 KR 20180001356
(71) Applicant: LG Household & Health Care Ltd., Seoul 03184 (KR)
(72) Inventor: KWON, Koo-Chul, Seoul 07795 (KR); SHIM, Woo-Sun, Seoul 07795 (KR); YEOM, Junseok, Seoul 07795 (KR); KO, Won-Jin, Seoul 07795 (KR); KANG, Nae-Gyu, Seoul 07795 (KR)
(74) Representative: Goddar, Heinz J.
(86) International application number: PCT/KR2018/016495
(87) International publication number: WO 2019/135528

(57) **Abstract**

The present invention relates to a powder complex for cosmetics and a cosmetic composition comprising thereof. More specifically, the present invention relates to a powder complex for cosmetics for improving easy-to-clean of the cosmetic powder with water repellency and a cosmetic composition comprising thereof. The powder complex for cosmetic of the present invention has a technical feature that the amphipathic copolymer having a specific structure is adsorbed on the surface of the hydrophobized powder. The cosmetic composition according to the present invention can exhibit an excellent effect of cleaning, with excellent makeup durability.

## Description

### [TECHNICAL FIELD]

The present application claims the priority based on Korean Patent Application No. 10-2018-0001356 filed on January 4, 2018, and the entire contents disclosed in the description and drawings of the corresponding application are incorporated in the present application.

The present invention relates to a powder complex for cosmetics and a cosmetic composition comprising thereof. More specifically, the present invention relates to a powder complex for improving easy-to-clean of a cosmetic powder with water repellency and a cosmetic composition comprising thereof.

### [BACKGROUND ART]

Powders mixed in cosmetic compositions such as body cosmetics including makeup cosmetics such as eye shadows, and the like, body powders, baby powders, etc., and cosmetics for blocking ultraviolet rays including suncream, sun sticks, etc., and the like, have been used as the surface is water repellent treated. Methods for water repellent treatment of powder surfaces are well known in the art. As one example, a method for imparting hydrophobicity to the surface of the powder by coating the surface of the powder using silicones such as higher fatty acids, higher alcohols, hydrocarbons, triglycerides, esters, silicone oils, silicone resins, etc., or fluorine compounds, and the like, is included.

However, when the hydrophobized powder is mixed, the makeup durability, but a problem in that easy-to-clean (rinsability) is lowered appears. It has excellent durability, but a problem in that cosmetics remain on the skin even after cleaning appears. When cosmetics remain on the skin, pores are blocked and therefore skin troubles are caused or a problem such as pigmentation, and the like, are caused, and thereby users experience great discomfort.

Accordingly, there is a continuing need for a cosmetic composition with excellent makeup durability and excellent easy-to-clean when cleaning.

### [DISCLOSURE]

### [TECHNICAL PROBLEM]

Accordingly, a problem to be solved by the present invention is to solve the aforementioned problems and to provide a powder complex for cosmetics for improving easy-to-clean when cleaning with excellent makeup durability, and a cosmetic composition comprising thereof.

Specifically, the present invention is to provide a powder used for a cosmetic composition, particularly, a powder complex for improving easy-to-clean of the hydrophobized powder and a cosmetic composition comprising thereof.

### [TECHNICAL SOLUTION]

To solve the above problems, the present invent ion provides a powder complex for cosmetics in which an amphipathic copolymer represented by the following Chemical formula (1) is adsorbed to a powder, and a cosmetic composition comprising thereof.

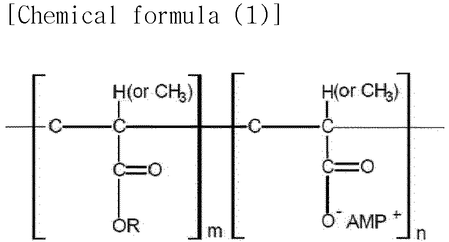

In the Chemical formula (1), R is a C₁∼C₈ aliphatic hydrocarbon group, and AMP is aminomethyl-2-propanol or amino-2-methyl-1,3-propandiol. In addition, m and n are an integer of 1 or more, respectively, and m + n < 1,000,000.

The R is a C1∼C8 aliphatic hydrocarbon group, and the aliphatic hydrocarbon group may be more specifically, a C1-C8 linear or branched, saturated or partially unsaturated, and chain or cyclic form, but not limited thereto. Preferably, it may be a linear saturated hydrocarbon group. The AMP is aminomethyl-2-propanol or amino-2-methyl-1,3-propanediol, and the m and n are an integer of 1 or more, respectively, and m + n <1,000,000. Preferably, the m and n may be an integer of 10 or more, respectively, and it may be m + n <100,000. In addition, the polymerization ratio of each monomer of the amphipathic copolymer is not limited thereto, but for an excel lent effect of the present invention, m:m may be determined among 1 : 1 < m : n < 1 : 100, and preferably, it may be 1 : 1 < m : n < 1 : 10. More preferably, m : n may be 3 : 7.

The inventors of the present invention have studied and tried to provide a powder for cosmetics with excellent water repel lency and improved easy-to-clean and a cosmetic composition comprising thereof. As a result, a powder complex in which the amphipathic copolymer represented by the Chemical formula (1) is adsorbed on the surface of a powder, in particular, a powder of which surface is already hydrophobized by a known water repellent treating agent was prepared. It has been found that a cosmetic composition comprising the powder complex obtained as above still has excellent water repellency and makeup durability when applying it to skin, and also when cleaning it using a known cleaning agent comprising water or surfactant, the easy-to-clean property is excellently improved, thereby completing the present invention.

Herein, 'powder' is preferably a powder used for cosmetics. The powder used for cosmetics may include inorganic powders such as titanium oxide, zirconium oxide, zinc oxide, cerium oxide, magnesium oxide, barium sulfate, calcium sulfate, calcium carbonate, magnesium carbonate, talc, mica, kaolin, sericite, white mica, synthetic mica, phlogopite, lepidolite, biotite, lithia mica, silicate, silica acid anhydride, aluminum silicate, magnesium silicate, calcium silicate, barium silicate, strontium silicate, tungstic acid metal salt, hydroxyapatite, vermiculite, haidilite, bentonite, montmorillonite, hectorite, zeolite, ceramic powder, calcium phosphate, dibasic, alumina, aluminum hydroxide, boron nitride, silica and complexes thereof, and the like, and organic powders such as polyamide, polyester, polyethylene, polypropylene, polystyrene, polyurethane, benzoguanamine, polymethylbenzoguanamine, polytetraflouroguanamine, polytetrafluoroethylene, polymethylmethacrylate, vinyl resin, urea resin, phenol resin, fluorine resin, silicon resin, acrylic resin, melamine resin, epoxy resin, polycarbonate resin, divinylbenzene styrene copolymer, copolymer consisting of two kinds or more of the monomers of the compound, celluloid, acetyl cellulose, cellulose, polysaccharide, protein, CI pigment yellow, CI pigment orange, CI pigment green, silk powder, nylon powder, 12 nylon, 6 nylon, acrylic powder, styrene acrylate copolymer, and the like, but not limited thereto. In addition, preferably, inorganic powders such as titanium dioxide, iron oxide red, iron sulfate, black iron oxide, zinc oxide, cerium oxide, cobalt titanate, magnesium oxide, zirconium oxide, talc, kaolin, sericite, white mica, phlogopite, synthetic mica, crimson mica, biotite, and the like may be used. More preferably, inorganic powders such as titanium dioxide, zinc oxide, iron oxide red, iron sulfate, black iron oxide, zinc oxide, cerium oxide, cobalt titanate, magnesium oxide, zirconium oxide, talc, kaolin, sericite, white mica, phlogopite, synthetic mica, crimson mica, biotite, and complexes thereof may be used. More preferably, titanium dioxide, zinc oxide, talc and mixed powders thereof may be used. In addition, the shape of the powder may be for example, a plate shape, a sphere shape, a porous sphere shape, a scaly shape, and the like, but the shape of the powder is not limited thereto, and the particle diameter, and the like are not particularly limited.

Herein, as the powder, a powder of which surface is hydrophobized may be used, and then, the amphipathic copolymer according to the present invention may provide a powder complex adsorbed on the surface of the hydrophobized powder. Herein, the 'hydrophobization' means powder processing known in the art to provide water repellency to the powder used for cosmetics. The hydrophobization may be performed by any known method without particular limitation, and for example, a method for treat ing the powder surface by at least one of water repellent treating agents selected from the group including silicon compound, alkyl silane compound, alkyl titanate compound, fluorine compound, amino acid compound and fatty acid compound, and the like may be exemplified. As the silicon compound, for example, a silicon compound having a hydrogen atom directly binding to the powder may be used, and for example, dimethicone, methicone, hydrogen dimethicone, trietoxycaprylsilane, acrylic silicon, cetyldimethicone copolymer, (dimethicone/methicone)copolymer, trietoxysilylethylpolydimethylsiloxyethyldimethicone, triethoxysilylethylpolydimethylsiloxyethylhexyldimethicone, (acrylate/acrylate tridecyl/methacrylate triethoxysilylpropyl/methacrylate dimethicone)copolymer, PEG-11 methyletherdimethicone, polyglyceryl-3 disiloxanedimethicone, (stearoxymethicone/dimethicone)copolymer, PEG/PPG-10/3 oleyletherdimethicone, trimethylsiloxysilicate, methylphenylsilicon, dimethicone copolymer, (dimethicone/vinyldimethicone)copolymer, (acrylate alkyl/dimethicone)copolymer, (aminomethylaminopropylmethicone/dimethicone)copolymer, and the like may be used, but not limited thereto. As the alkyl silane compound, a known silane coupling agent may be used, and for example, dimethylmethoxysilane, methyltrimethoxysilane, dimethyldiethoxysilane, methyltrietoxysilane, diphenylethoxysilane, phenyltriethoxysilane, 3-glycidoxypropyltriethoxysilane, 5,6-epoxyhexyltriethoxysilane, 2-(3,4-epoxycyclohexyl)ethyltriethoxysilane, 3-acryloxypropyltriethoxysilane, 3-methacryloxypropyltriethoxysilane, N-(2-aminoethyl)-3-aminopropyltriethoxysilane, 3-aminopropyltriethoxysilane, 3-mercaptopropylmethyldiethoxysilane, n-octyltriethoxysilane, and the like may be used, but not limited thereto. As the alkyl titanate compound, for example, isopropoxytitaniumtristearate, triisopropoxytitaniumisostearate, isopropoxytitaniumtripalmitate, isopropoxytitaniumtrimyristate, and the like may be used, but not limited thereto. As the fluorine compound, for example, trifluoropropyltrimethoxysilane, trifluoropropyltriethoxysilane, tridecafluorooctyltrimethoxysilane, and the like may be used, but not limited thereto. The amino acid compound may include for example, N-acyl amino acid metal salt and the like, and the precursor of the N-acyl amino acid metal salt, N-acyl amino acid may include for example, N-acyl-N-methyl-glycine, N-acyl-N-methyl-b-alanine and N-acyl-L-glutamate, and the like, but not limited thereto. As the fatty acid compound, for example, as the fatty acid, aluminum myristate, sodium myristate, calcium myristate, magnesium stearate, aluminum stearate, sodium stearate, calcium stearate, and palmitic acid, dimethiconol stearate, dimethiconol hydroxystearate, and the like may be used, but not limited thereto. In particular, When the powder surface is hydrophobized so as to have a hydrocarbon chain, the adsorption with the amphipathic copolymer according to the present invention may be increased, and therefore it is preferable to hydrophobize the surface of the powder so as to have a hydrocarbon chain. The hydrophobized powder is prepared and used, or a hydrophobized powder prepared and commercially available may be used.

The powder complex according to the present invention may be a form in which the amphipathic copolymer of Chemical formula (1) is adsorbed on the surface of the powder (preferably, hydrophobized powder). In the specification of the present invention, the term used to show that the amphipathic copolymer is 'adsorbed' to the powder, 'adsorb' means that the amphipathic copolymer is attached, bound, coated or covered to the powder, and the form of adsorption is not particularly limited, as long as it is a form to achieve the effect of the present invention. As the amphipathic copolymer is attached on the surface of the powder, or on the hydrophobized surface of the powder in case of using the hydrophobized powder, the water repellent effect of the powder is maintained, and it may be easily removed when cleaning.

Herein, the attached amount of the amphipathic copolymer to the powder may be the amphipathic copolymer of 5 to 30 % by weight, based on the total weight of the powder. Preferably, it may be 8 to 23 % by weight. In the specification of the present invention, when the powder is a hydrophobized powder, the attached amount shows the amphipathic copolymer of 5 to 30 % by weight based on the total weight of the powder before hydrophobized. When the weight of the attached amphipathic copolymer is 5 to 30 % by weight based on the total mass of the powder, a powder complex with excellent water repellency and improved easy-to-clean may be provided. When the attached amount is less than 5 % by weight, the water repel lent effect of the hydrophobized powder is large and therefore dispersion in an aqueous phase is difficult, and it is impossible to exhibit excellent cleaning ability, and when it is over 30 % by weight, a powder in which the amphipathic copolymer is attached on the surface is not prepared.

Herein, the amphipathic copolymer of Chemical formula (1) may preferably comprise at least one of amino-2-methyl-1,3-propanediol-acrylate copolymer and aminomethyl-2-propanol-acrylate copolymer, and more preferably, amino-2-methyl-1,3-propanediol-acrylate copolymer, aminomethyl-2-propanol-acrylate copolymer alone or mixture thereof may be used. The inventors of the present invention have confirmed that a cosmetic composition comprising a powder complex for cosmetics prepared using the amino-2-methyl-1,3-propanediol-acrylate copolymer, aminomethyl-2-propanol-acrylate copolymer alone or mixture thereof as the amphipathic copolymer according to the present invention has excellent makeup durability and significantly improves a problem of remaining in skin after cleaning.

In the powder complex for cosmetics according to the present invention, attachment of the amphipathic copolymer to the powder may be performed according to a powder coating method known in the art. Preferably, it may be prepared by a wet process or dry process.

In the wet process, a process of dispersing a powder (preferably, hydrophobized powder) and an amphipathic copolymer in an appropriate solvent, and then stirring with a homogenizing mixer, and adsorbing the amphipathic copolymer on the surface of the powder may be progressed. The stirring may be performed for example, by a mixer such as reactor with stirring wings or disper mixer, Henschel mixer, homo mixer, kneader, and the like. In addition, the stirring may be performed at 1,000 to 10,000 rpm, and for homogeneous adsorption to the powder surface, preferably, it may be progressed at 3,000 to 7,000 rpm, and more preferably, it may be progressed at 5,000 to 6,000 rpm. Moreover, the stirring may be performed for 30 minutes to 3 hours, and preferably, it may be performed for 1 hour to 2 hours. The appropriate solvent is a solvent known to be used in the wet process, and it maybe used in a range that does not impair the object of the present invention, and for example, alcohol-based organic solvents such as methanol, ethanol, propanol, isopropanol, butanol, benzyl alcohol, propylene glycol, ethylene glycol, etc., ketone-based organic solvents such as methylethylketone, methylisobutylketone, cyclohexanone or methylcyclohexanone, etc., ester-based organic solvents such as methyl acetate, ethyl acetate, propyl acetate, butyl acetate, ethyl formate, propyl formate, butyl formate or ethyl lactate, etc., amide-based organic solvents such as dimethylformamide, dikethylacetamide or n-methylpyrrolidone, etc., ether-based organic solvents such as propylene glycol, monomethyl ether, dioxane, tetrahydrofuran, ethylene glycol dimethylether or dimethylether , etc., and the like may be used, but not limited thereto. Among then, in particular, it is preferable to use alcohol-based solvents such as ethanol, isopropyl alcohol, isobutanol, and the like, and more preferably, ethanol may be used. When the alcohol-based solvent, particularly, ethanol is used as the solvent, the excellent stability of the powder complex for cosmetics of the present invention may be shown.

In the dry process, it may be performed by a method of dissolving an amphipathic copolymer in an appropriate solvent to prepare a solution, and then spraying it to a powder (preferably, hydrophobized powder). As the appropriate solvent for the dry process, a known solvent used for the dry process may be used in the range that does not impair the object of the present invention, and for example, alcohol-based organic solvents such as methanol, ethanol, propanol, isopropanol, butanol, benzyl alcohol, propylene glycol, ethylene glycol, etc., ketone-based organic solvents such as methylethylketone, methylisobutylketone, cyclohexanone or methylcyclohexanone, etc., ester-based organic solvents such as methyl acetate, ethyl acetate, propyl acetate, butyl acetate, ethyl formate, propyl formate, butyl formate or ethyl lactate, etc., amide-based organic solvents such as dimethylformamide, dikethylacetamide or n-methylpyrrolidone, etc., ether-based organic solvents such as propylene glycol, monomethyl ether, dioxane, tetrahydrofuran, ethylene glycol dimethylether or dimethylether, etc., and the like may be used, but not limited thereto. Among then, in particular, it is preferable to use alcohol-based solvents such as ethanol, isopropyl alcohol, isobutanol, and the like, and more preferably, alcohol may be used. When the alcohol-based solvent, particularly, ethanol is used as the solvent, the excellent stability of the powder complex for cosmetics of the present invent ion may be shown. In the solution in which the amphipathic copolymer is dissolved, it is preferable that the concentration of the amphipathic copolymer is determined from 5% to 10%, and more preferably, it may be 7.5%. When the dry process is progressed with the solution at the above concentration, a powder complex for cosmetics showing the desired effect of the present invention may be prepared, and when using it at a concentration other than the range, a problem in that the stability of the powder complex is deteriorated may be shown.

The present invention may provide a cosmetic composition comprising the powder complex for cosmetics according to the present invention. The cosmetic composition according to the present invention may show the effect of excellent makeup durability and significantly improving the problem in that cosmetics remain in skin when cleaning. The powder complex for cosmetics may be comprised by 1 to 30 % by weight, preferably, 5 to 25 % by weight, more preferably, 20 % by weight, based on the total weight of the cosmetic composition, but not limited thereto.

Herein, the cosmetic composition may be prepared in a formulation of foundation, solution, external ointment, cream, foam, nutrient cosmetic water, flexible cosmetic water, pack, makeup base, primer, essence, sunscreen cream, sun oil, sunscreen stick, suspension, emulsion, paste, gel, lotion, oil, spray, and the like, and the cosmetic may be prepared in various forms such as emulsion form, cream form, stick form, solid form, multilayer separation form, and the like, but not limited thereto. In particular, the cosmetic composition according to the present invention may show an excellent effect in the washability, rinsability, easy-to-clean, and the like, compared to conventional cosmetics, when it is mixed to a formulation for which skin residues of cosmetics after washing are a problem, particularly, cosmetics for blocking ultraviolet rays in which components for blocking ultraviolet rays, liquid foundation cosmetics, makeup base or primer.

When preparing the cosmetic composition according to the present invention, the powder complex for cosmetics according to the present invent ion may be prepared by being dispersing in an aqueous phase or oil phase by a known method. The form of the comprised powder complex for cosmetics according to the present invention is not greatly limited, but as one example, the powder complex may be prepared as comprised in a form of dispersion.

The cosmetic composition according to the present invention may further comprise at least one of a wetting agent and a dispersing agent, and the wetting agent is not particularly limited as long as any wetting agent used for the cosmetic composition, but for example, it may include 1,3-butylene glycol, 1,2-hexanediol, polyethylene glycol, polypropylene glycol, polyethylene glycol, polypropylene glycol, polybutylene glycol-8/5/3 glycerin, and the like. Preferably, glycerin may be used as the wetting agent. The content of the wetting agent may be 10 to 30 % by weight, preferably, 20 % by weight, based on the total weight of the cosmetic composition. As the dispersing agent, a known dispersing agent used for the cosmetic composition may be used, and as long as using one with 7.0 or more of HLB (Hydrophil-Lipophile balance), the kind is not particularly limited in the range that does not impair the object of the present invention. The content of the dispersing agent may be 5 to 15 % by weight, preferably, 10 % by weight, based on the total weight of the cosmetic composition. When the wetting agent and the dispersing agent are comprised in the above content, respectively, it may show the excellent stability of the cosmetic composition and the effect of certain cosmetic composition.

When the cosmetic composition according to the present invention is prepared as a cosmetic for blocking ultraviolet rays, the cosmetic composition for blocking ultraviolet rays may further comprise a dispersing agent and/or a wetting agent, particularly, and in particular, it may be prepared in a form of dispersion.

In addition, the cosmetic composition for blocking ultraviolet rays according to the present invention may comprise an inorganic ultraviolet blocking agent, organic ultraviolet blocking agent or mixture thereof. Herein, the inorganic ultraviolet blocking agent, organic ultraviolet blocking agent or mixture thereof may be comprised in a content of 1 to 40 % by weight, preferably, 10 to 30 % by weight, more preferably, 20 to 25 % by weight, based on the total weight of the cosmetic composition, but not limited thereto.

Herein, as the "inorganic ultraviolet blocking agent' , inorganic powders showing the ultraviolet blocking effect may be used, and in particular, inorganic powders that are zinc oxide, titanium dioxide or mixture thereof may be used. The inorganic powder used as the inorganic ultraviolet blocking agent is preferably comprised by being prepared as the powder complex according to the present invention on the purpose of the present invention. When the inorganic ultraviolet blocking agent is prepared and comprised as the powder complex according to the present invention, the present invention may provide a cosmetic composition for blocking ultraviolet rays showing the stable effect of blocking ultraviolet rays. More specifically, the present invention may provide a cosmetic composition for blocking ultraviolet rays which maintains the effect of blocking ultraviolet rays in everyday life and improves easy-to-clean when cleaning.

Herein, as the 'organic ultraviolet blocking agent' , for example, at least one selected from ethylhexylsalicylate, ethylhexylpalmitate, octocrylene, phenylbenzimidazole sulfonic acid, isoamyl-p-methoxycinnamate, homosalate, butylmethoxydibenzoylmethane, ethylhexylmethoxycinnamate, 4-methylbenzylidene camphor and bis-ethylhexyloxyphenolmethoxyphenyltriazine, and the like, but not limited thereto.

The inventors of the present invention have confirmed that the cosmetic composition for blocking ultraviolet rays according to the present invention shows excellent ultraviolet blocking effect and easy-to-clean compared to the cosmetic composition for blocking ultraviolet rays in which the present invention is not applied. Specifically, it has been confirmed that the cosmetic composition in contrast to the cosmetic composition for blocking ultraviolet rays according to the present invention has the initial ultraviolet blocking effect similar thereto after applying to skin, but while as time passes, the ultraviolet blocking effect of the present invention is maintained, the ultraviolet blocking effect of the contrast cosmetic composition is reduced. In addition, it has been confirmed that the contrast cosmetic composition remains in skin, but the cosmetic composition for blocking ultraviolet rays according to the present invention is clearly cleaned, when it is applied to skin and dried for 1 hour, and then washed with water using a cleaning agent. Through this, it has been confirmed that a cosmetic composition for blocking ultraviolet rays with excellent ultraviolet blocking effect and easy-to-clean may be provided by applying the present invention.

The cosmetic composition for blocking ultraviolet rays according to the present invention may exhibit an effect of excellent makeup durability and greatly improving easy-to-clean, due to excellent water resistance, oil resistance, sebum resistance, and the like. Conventionally, components mixed to cosmetic compositions for blocking ultraviolet rays might cause various kinds of problems such as skin trouble, weakly, to skin cancer, severely, when remained in skin after cleaning. Accordingly, when cleaning of the cosmetic composition is facilitated, there was a problem in that the makeup durability was deteriorated, and as time passed after applying the cosmetic, a problem in that the effect of blocking ultraviolet rays was deteriorated was caused. When applying the present invention, a cosmetic composition showing both excellent makeup durability and easy-to-clean, particularly, a cosmetic composition for blocking ultraviolet rays in which these problems are greatly improved may be provided.

The cosmetic composition according to the present invention may be prepared by mixing components commonly mixed to the cosmetic composition, for example, surfactant, emulsifier, gelling agent, polymer, beauty component, ultraviolet blocking component, moisturizer, pigment, preservative, flavor, and the like, in an appropriate amount in the range that does not impair the effect of the present invention, in addition to the aforementioned components, by a common method.

### [ADVANTAGEOUS EFFECTS]

The powder complex for cosmetics of the present invention can exhibit an effect of being easily removed when cleaning by a surfactant and the like, maintaining an effect of water repellent treatment of the powder.

The cosmetic composition comprising the powder complex for cosmetics according to the present invention can have excellent makeup durability and be easily cleaned without requiring excessive cleansing when cleaning, and can exhibit an effect of reducing problems such as skin trouble, and the like, by significantly improving the skin residue of cosmetics.

The cosmetic composition for blocking ultraviolet rays according to the present invention can exhibit an excellent ultraviolet-protecting effect and easy-to-clean.

### [BRIEF DESCRIPITON OF THE DRAWINGS]

The following drawings attached to the present specification are intended to illustrate preferable examples, and play a role of further understanding the technical spirits of the present invention together with the aforementioned contents of the invention, and therefore the present invention should not be interpreted as limited only to those described in those drawings.

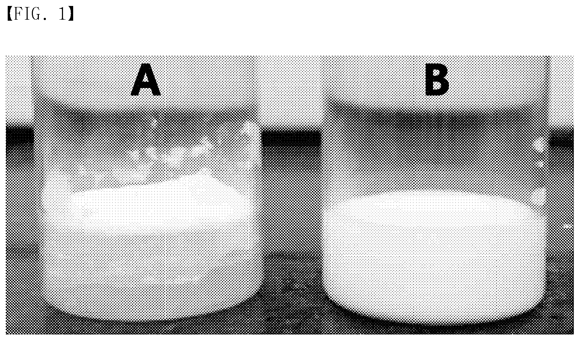

FIG. 1 is the result of comparing the water dispersity of titanium dioxide surface-treated with 17% aluminum stearate (A, left) and the powder complex in which an amphipathic copolymer is attached on titanium dioxide surface-treated by 17% aluminum stearate by applying the present invention (B, right).

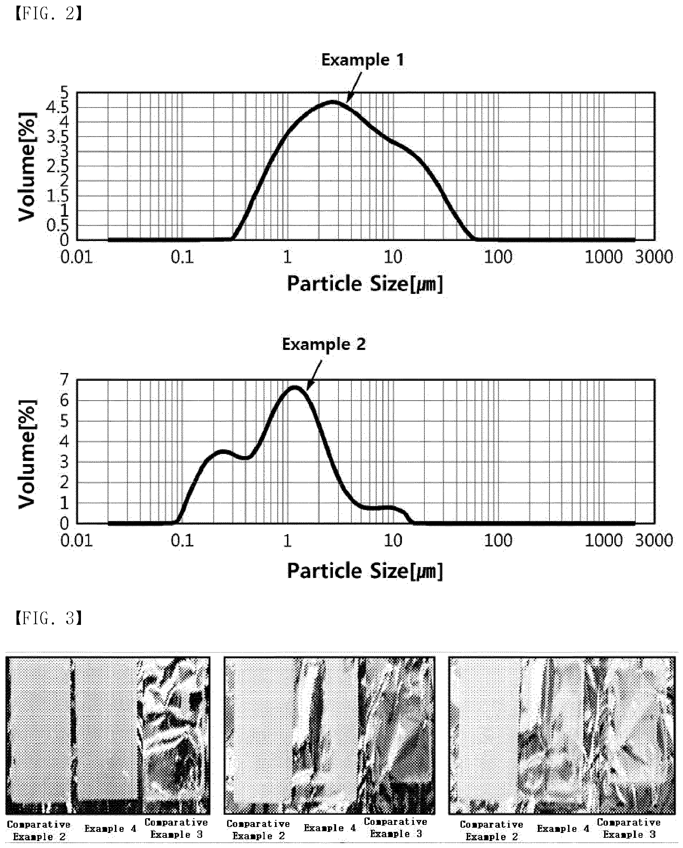

FIG. 2 shows the result of the dynamic light scattering particle size distribution of Example 1 (A) and Example 2 (B) prepared according to the present invention as a graph. The y-axis of the graph represents the volume (%) and the x-axis represents the particle size (µm).
FIG. 3 shows the result of comprising the difference in water repellency and cleaning power of Example 4, Comparative example 2 and Comparative example 3. In each photograph, the left shows Comparative example 2, and the center shows Example 4, and the right shows Comparative example 3. The left photograph shows the photograph after stirring at 700 rpm for 30 minutes, and the center photograph shows the photograph after adding the cleaning agent and then stirring at 700 rpm for 120 minutes.

### [BEST MODE]

Hereinafter, to help understanding of the present invention, it will be described in detail by examples. However, the examples according to the present invention may be modified in many different forms, and the scope of the present invention should not be interpreted as being limited to the following examples. The examples of the present invention are provided to more completely describe the present invention to those skilled in the art.

### Preparation of powder complexes for cosmetics - preparation of Example 1 and Example 2

50% ethanol dispersion of an amphipathic copolymer (trade name: MIHAPOL PAU-50, Miwon Commercial Co., Ltd.) of 600 parts by mass was added to a homo mixer, and diluted in ethanol of 400 parts by mass, and then titanium dioxide surface-treated by 17% aluminum stearate (trade name: MT-100TV, TAYCA corp.) of 1000 parts by mass was added and stirred at 6,000 rpm for 60 minutes. Then, the reactants were centrifuged at 5,000 rpm for 10 minutes to separate non-reacted amphipathic copolymer. Then, it was purified using purified water at 5,000 rpm for 10 minutes twice to prepare a powder complex in which the amphipathic copolymer was adsorbed in an amount of 23 % by weight based on the total weight (Example 1). The weight of the powder based on the total weight of the powder complex was 35% by weight.

By the same process as above, using zinc oxide (trade name: SUNZnO-NAS, Sunjin Beauty Science), a powder complex in which the amphipathic copolymer was adsorbed in an amount of 8% by weight based on the total weight of the powder (Example 2) was prepared. The weight of the powder based on the total weight of the powder complex was 65% by weight.

### [Comparative evaluation of water dispersity of Example 1]

The water dispersity of the powder complex prepared above (Example 1) and 17% aluminum stearate untreated with the amphipathic copolymer was comparatively experimented, and the result was shown in FIG. 1.

### [Analysis of particle size distribution of Example 1 and Example 2]

The analysis of the particle size of the powder complexes of Example 1 and Example 2 prepared above was performed. The result of the analyzed particle size distribution of the powder complexes using a dynamic light scattering particle size analyzer was shown in FIG. 2.

### Preparation of cosmetic compositions

### [Preparation of suncream in an O/W formulation - preparation of Example 3 and Comparative example 1]

Oil-in-water (O/W) suncream of Example 3 and Comparative example 1 was prepared by the composition of the following Table 1. The aqueous phase among the components shown in the following table was prepared by uniformly dissolving at a room temperature. The oil phase among the components shown in the following table was prepared by uniformly dissolving at 60°C, and then mixing the aqueous phase, to prepare cosmetic compositions.

**[Table 1]**

| | Raw material | Example 3 | Comparative example 1 |
|---|---|---|---|
| 1 | Neopentyl Glycol Dicaprate | 5.0 g | 5.0 g |
| 2 | Cetyl Ethylhexanoate | 5.0 g | 5.0 g |
| 3 | Dimethicone | 3.0 g | 3.0 g |
| 4 | Polyglyceryl-3 Methylglucose Distearate | 3.0 g | 3.0 g |
| 5 | PEG-40 Stearate | 0.5 g | 0.5 g |
| 6 | MT-100TV | - | 6.8 g |
| 7 | Dipropylene Glycol | 4.0 g | 4.0 g |
| 8 | Hydroxyethyl Acrylate/Sodium Acryloyldimethyl Taurate Copolymer (and) squalane (and) polysorbate 60 | 1.0 g | 1.0 g |
| 9 | 1,2-Hexandiol | 2.0 g | 2.0 g |
| 10 | Purified water | Remaining amount | Remaining amount |
| 11 | Example 1 | 16.0 g | - |
| Total | | 100.0g | 100.0g |

### Evaluation of cleaning power of Example 3 and Comparative example 1

Using the oil-in-water (O/W) suncream of Example 3 and Comparative example 1 prepared as above, the cleaning power was evaluated by the following method.

After applying 50µl of each cosmetic composition of Example 3 and Comparative example 1 prepared to skin, respectively, it was dried for 1 hour. Then, by rubbing with running water by hand 5 times and washing, a phenomenon in that each composition was removed was confirmed. The result of confirming the degree of cleaning using an ultraviolet light mode of JANUS (PIE Co., Ltd.) was shown in the following Table 2.

**[Table 2]**

| | Cleaning power |
|---|---|
| Example 3 | ⊚ |
| Comparative example 1 | ○ |

| | |
|---|---|
| ⊚ : Completely cleaned ○ : Excellent cleaning power Δ : Normal cleaning power X : Not cleaned | |

As could be confirmed in the Table 2, it could be confirmed that the washability of the cosmetic composition was improved by applying the present invention.

### [Preparation of suncream in a W/O formulation - preparation of Example 4 and Comparative example 2]

Water-in-oil (W/O) suncream of Example 4 and Comparative examples 2 and 3 was prepared by the composition of the following Table 3. The aqueous phase among the components shown in the following table was prepared by uniformly dissolving at a room temperature. The oil phase among the components shown in the following table was prepared by uniformly dissolving at 80°C, and then mixing the aqueous phase, to prepare cosmetic compositions.

**[Table 3]**

| | Raw material | Example 4 | Comparative example 2 | Comparative example 3 |
|---|---|---|---|---|
| 1 | Magnesium Sulfate | 0.5 g | 0.5 g | 0.5 g |
| 2 | Tromethamine | 2.0 g | 2.0 g | 2.0 g |
| 3 | 1,3-Butylene glycol | 10.0 g | 10.0 g | 10.0 g |
| 4 | Phenylbenzimidazole Sulfonic Acid | 4.0 g | 4.0 g | 4.0 g |
| 5 | 1,2-Hexanediol | 1.0 g | 1.0 g | 1.0 g |
| 6 | Purified water | Remaining amount | Remaining amount | Remaining amount |
| 7 | Example 1 | 12.0g | - | - |
| 8 | Example 2 | 21.0g | - | - |
| 9 | TXD555-AQ | - | - | 9.0 g |
| 10 | SUNCLEAR-Z75AQS | - | - | 17.5 g |
| 11 | Cyclopentasiloxane | 31.5 g | 13.5 g | 31.5 g |
| 12 | Isostearic acid | 0.5 g | 0.5 g | 0.5 g |
| 13 | Cetyl Ethylhexanoate | 7.0 g | 7.0 g | 7.0 g |
| 14 | Disteardimonium Hectorite | 1.0 g | 1.0 g | 1.0 g |
| 15 | PEG-10 dimethicone | 4.0 g | 4.0 g | 4.0 g |
| 16 | Sorbitan Sesquiisostearate | 0.5 g | 0.5 g | 0.5 g |
| 17 | MT-100TV | - | 5.0 g | - |
| 18 | SUNZnO-NAS | - | 13.0 g | - |
| Total | | 100.0 g | 100.0 g | 100.0 g |

### Evaluation of cleaning power of Example 4 and Comparative example 2

Using the water-in-oi 1 (W/O) suncream of Example 4 and Comparative examples 2 and 3 prepared as above, the cleaning power was evaluated by the following method.

After applying 50µl of each cosmetic composition of Example 4 and Comparative examples 2 and 3 prepared to skin, respectively, it was dried for 1 hour. Then, by rubbing with running water by hand 5 times and washing, a phenomenon in that each composition was removed was confirmed. The result of confirming the degree of cleaning using an ultraviolet light mode of JANUS (PIE Co., Ltd.) was shown in the following Table 4.

**[Table 4]**

| | Cleaning power |
|---|---|
| Example 4 | ⊚ |
| Comparative example 2 | Δ |

| | |
|---|---|
| ⊚ : Completely cleaned ○ : Excellent cleaning power Δ : Normal cleaning power X : Not cleaned | |

As could be confirmed in the Table 4, it could be confirmed that the washability of the cosmetic composition was improved by applying the present invention.

### Evaluation of water repellency and cleaning powder of Example 4 and Comparative example 2 to Comparative example 3

By evaluating the water repellency and cleaning power of the cosmetic compositions of Example 4 and Comparative example 2 to Comparative example 3 prepared above by the following method, the result was shown in FIG. 3 and the following Table 5.

After applying 1g of each cosmetic composition of Example 4 and Comparative examples 2 and 3 prepared on a glass plate, respectively, it was dried for 1 hour. Then, the degree of cleaning of the cosmetic compositions was confirmed by stirring at 700 rpm in 25°C water. Then, a cleaning agent was added and it was stirred at 700 rpm for 120 minutes. At that time, in 30 minutes and 120 minutes after adding the cleaning agent, the degree of cleaning of each cosmetic composition was confirmed.

**[Table 5]**

| 700 rpm stirring | In 30 minutes | In 30 minutes after adding the cleaning agent | In 120 minutes after adding the cleaning agent |
|---|---|---|---|
| Comparative example 2 | X | X | X |
| Example 4 | X | ○ | ⊚ |
| Comparative example 3 | ○ | ⊚ | ⊚ |

| | | | |
|---|---|---|---|
| ⊚ : Completely cleaned ○ : Excellent cleaning power Δ : Normal cleaning power X : Not cleaned | | | |

As could be confirmed in the Table 5 and FIG. 3, it could be confirmed that when applying the present invention, the water repellency was maintained in everyday life and therefore the makeup durability was excellent, whereas the easy-to-clean and washability was excellent by a cleaning agent.

## Claims

1. A powder complex for cosmetics in which an amphipathic copolymer represented by the following Chemical formula (1) is adsorbed to a powder. 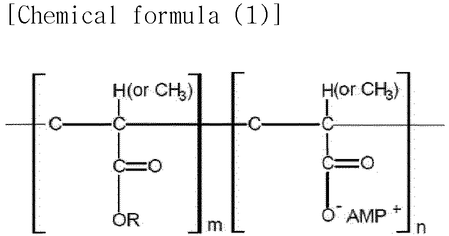 (In the Chemical formula (1), R is a C₁∼C₈ aliphatic hydrocarbon group, and AMP is aminomethyl-2-propanol or aminomethyl-2-methyl-1,3-propanediol. In addition, m and n are an integer of 1 or more, respectively, and m + n < 1,000,000.)

2. The powder complex for cosmetics according to claim 1, wherein the powder is hydrophobized powder, and the amphipathic copolymer is adsorbed on the surface of the hydrophobized powder.

3. The powder complex for cosmetics according to claim 2, wherein the hydrophobization is performed by a water repellent treatment agent comprising silicone compound, alkyl silane compound, alkyl titanate compound, fluorine compound, amino acid compound, fatty acid compound and mixtures thereof.

4. The powder complex for cosmetics according to claim 1, wherein the amphipathic copolymer is adsorbed on the surface of the powder.

5. The powder complex for cosmetics according to claim 4, wherein the adsorption amount of the amphipathic copolymer is the amphipathic copolymer of 5 to 30 % by weight based on the total weight of the powder.

6. The powder complex for cosmetics according to claim 1, wherein the amphipathic copolymer is at least one of amino-2-methyl-1,3-propanediol-acrylate copolymer and aminomethyl-2-propaneol-acrylate copolymer.

7. The powder complex for cosmetics according to claim 1, wherein the powder complex for cosmetics is prepared by a wet process or dry process.

8. A cosmetic composition comprising the powder complex for cosmetics according to any one of claims 1 to 7.

9. The cosmetic composition according to claim 8, wherein the cosmetic composition further comprises at least one of a wetting agent and a dispersing agent.

10. The cosmetic composition according to claim 8, wherein the cosmetic composition is a cosmetic composition for blocking ultraviolet rays.
